# EUROPEAN PATENT SPECIFICATION

(11) **EP 4 215 230 B1**
(45) Date of publication and mention of the grant of the patent: **09.09.2026**
(21) Application number: 22305067.5
(22) Date of filing: 24.01.2022
(51) Int. Cl.: A61M 5/315

(54) **GLIDING PERFORMANCES IMPROVED THROUGH TRIM RIB DIMENSIONAL GEOMETRY/SHAPE**
DURCH TRIMMRIPPENGEOMETRIE/FORM VERBESSERTE GLEITLEISTUNG
PERFORMANCES DE GLISSEMENT AMÉLIORÉES GRÂCE À LA GÉOMÉTRIE/FORME DIMENSIONNELLE DE NERVURE DE GARNITURE

(43) Date of publication of application: 26.07.2023
(60) Divisional application: 26176454.2 / 4 782 032
(73) Proprietor: Becton Dickinson France, 38800 Le Pont-de-Claix (FR)
(72) Inventor: RIVE, Adrien, 38100 Grenoble (FR); RODRIQUEZ SAN JUAN, Nestor, Hamburg, NJ 07419 (US); ROSSITTO, Emmanuela, 38120 Saint Egreve (FR); MEYNIER, Chantal, 38600 Fontaine (FR); PERRIN, Eloïse, 38320 Eybens (FR)
(74) Representative: Germain Maureau

(56) References cited:
- WO-A1-2017/123838
- WO-A1-99/44659
- US-A1- 2018 325 728

## Description

### BACKGROUND OF THE INVENTION

### Field of the Invention

The present invention is directed to a stopper or a series of stoppers for use with injection devices, such as syringes, cartridges, and/or auto-injectors, having improved gliding performance by controlling the dimensional shape and/or geometry of a trim member on the stopper.

### Description of Related Art

Devices for automatic injection of a product, also called auto-injectors, are widely used in medical fields where the treatment of pathology requires daily injections, such as the treatment of some diabetes or arthritis, and where patients often perform these injections on their own. As patients are not professional healthcare workers, the whole process has been designed to be as automated as much as possible so that the patient does not have to make decisions during the injection. Auto-injectors usually comprise, on one hand, a container having a needle and filled with the product to be injected, such as a prefilled syringe, for example, or a cartridge, and on the other hand, a motor part, in other words, a part comprising the various systems which will trigger the insertion of the needle, realize the injection, and potentially activate a protection system at the end of injection.

Syringes, either prefilled or prefillable, auto-injectors, and other types of injection devices, often require slow and controlled initiation and maintenance of sliding movement of one surface over another surface. It is well known that two stationary surfaces having a sliding relationship often exhibit sufficient resistance to the initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose". Often times, this breakout or breakloose is partly caused by a concentration of stress between the trim member of a stopper, (i.e., the location where the individual stoppers are trimmed from the molding sheet and which typically has the largest diameter) that is in contact with the inner surface of the syringe barrel. "Breakout force" refers to the force required to overcome static friction between surfaces of a syringe assembly that has been previously moved in a sliding relationship, but has been stationary ("parked" or not moved) for a short period of time (for example, milliseconds to hours). A less well known but important frictional force is "breakloose force", which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Another important feature of an injection device is that the stopper should have good gliding properties i.e., a low "gliding force" to move the stopper within the container. The gliding . force of a stopper within either a filled or empty syringe is directly related to the main key product parameter of the syringe, the injection time of the drug contained in the syringe. This key product parameter is especially important for syringes used in auto-injectors, as it must comply with a defined injection time. Some stopper designs show a high variability regarding their breakloose and gliding performances which are linked to the diameter, shape, and eccentricity of the trimming surfaces.

A conventional approach to overcoming breakout, breakloose, and gliding forces has been the application of a lubricant to a surface interface. The use of a lubricant will enhance or reduce the gliding forces needed to move the stopper within the syringe barrel. Common lubricants used are silicone or hydrocarbon oils.

The use of some lubricants, especially when used with high-value biotech drugs, can have deleterious effects on these drugs. Thus, there is a need in the art to consistently control and/or reduce the gliding forces required to move a stopper within an auto-injector, syringe, and the like, while consistently controlling and/or reducing the amount of lubricant needed to achieve these reduced gliding forces. Patent documents WO 2017/123838 A1, WO 99/44659 A1, US 2018/325728 A1 disclose features falling under the preamble of claim 1.

### SUMMARY OF THE INVENTION

In accordance with one aspect, the present disclosure is directed to a stopper adapted for attachment with a plunger rod for use within a syringe barrel. The stopper comprises a main body defining an open rearward (proximal) end, a closed front (distal) end, and a cylindrical sidewall extending between the open proximal end and the closed distal end. The open proximal end is adapted to receive a distal forward end attachment portion of the plunger rod. At least one rib extends radially outward around a perimeter of the main body. This at least one rib is configured for forming an active seal with the syringe barrel. The stopper further includes a trim member extending radially outward around a perimeter of the main body at an interface between the closed distal end and the open proximal end. The trim member is the location where the individual stoppers are trimmed from the molding sheet. The trim member typically has the largest diameter of the stopper and thus is also in contact with the inner surface of the syringe barrel. The trim member is formed by controlling the geometry/shape, size, and diameter of the trim member with respect to the diameter of the stopper itself in order to reduce the gliding forces of the stopper within the barrel.

The stopper closed distal end has a first diameter and the trim member has a distally extending portion having a second diameter, wherein the second diameter is equal to or greater than the first diameter. According to the invention second diameter is approximately 2.3-2.8% greater than the first diameter.

According to one embodiment, the trim member can be located between the closed distal end and the at least one rib. Typically the stopper is molded in two parts and the trim member is located at the interface of the closed distal end and the open proximal end. A barrier film may be positioned on and/or adjacent to the closed front end.

In order to control the shape, geometry, and/or diameter of the trim member, the trim member is formed with a cutting tool, as opposed to a molding tool to form the stopper main body. The cutting tool can be a pre-stretched cutting die. Alternatively, the cutting tool can be a progressive cutting-edge die, which is a cutting tool that uses a wavy cutting edge engaging progressively on the rubber instead of a one-cut stroke die, which is prone to rubber displacement due to compressive stresses and the Poisson coupling effect. It can be appreciated that other types of cutting tools can be used to separate the stopper from the molding sheet, for example with laser or water jet cutting.

In accordance with another aspect, the present invention is directed to a method for forming consistent gliding forces for a series of stoppers. The method comprises providing a series of stoppers, each of the stoppers having a main body defining an open proximal end, a closed distal end, and a cylindrical sidewall extending between the open proximal end and the closed distal end. The open proximal end is adapted to receive a front distal end attachment portion of the plunger rod. At least one rib extends radially outward around a perimeter of the main body. The at least one rib is configured for forming an active seal with the syringe barrel and a trim member extends radially outward around a perimeter of the main body. The trim member has a diameter preferably configured for reducing gliding forces of the stopper within the barrel, wherein a shape, geometry, and/or width of the trim member of each of the series of stoppers is controlled by using a cutting tool, as opposed to a molding tool, to form the stopper main body. The cutting tool can be a pre-stretched cutting die, a progressive cutting-edge die, or any other well-known cutting tool.

Each of the stoppers are designed to have a first diameter and the trim member of each of the stoppers are designed to have a distally extending portion having a second diameter, wherein the second diameter is greater than the first diameter. According to the invention, the second diameter can be approximately 2.3-2.8% greater than the first diameter. The method may optionally include applying a barrier film on and/or adjacent to the closed front end of each of the stoppers, such that the barrier film extends to the trim member, and removing the excess barrier material from the stoppers.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
Fig. 1 is a side view of a stopper adapted for attachment with a plunger rod for use within a syringe barrel in accordance with an embodiment of the present invention.
Fig. 2 is a top perspective view of the stopper of Fig. 1 in accordance with an embodiment of the present invention.
Fig. 3 is a cross-sectional side view of the stopper of Fig. 1 in accordance with an embodiment of the present invention.
Fig. 4 is a graph illustrating the gliding performance of various stoppers, batches "a", "b", and "c", having a trim member in accordance with an embodiment of the invention.
Fig. 5A is a side view of a portion of the stopper of Fig. 1 wherein the distal, closed front end of the stopper has been removed in accordance with an embodiment of the present invention.
Fig. 5B is a side view of a portion of the stopper of Fig. 1 wherein the distal, closed front end and the trim member of the stopper have been removed in accordance with an embodiment of the present invention.
Fig. 5C is a side view of a comparative stopper similar to the stopper of Fig. 1, but wherein no trim member is present.
Fig. 5D1 is a graph similar to Fig. 4 and shows the gliding performances of stopper batches a, b, and c, having a trim member in accordance with an embodiment of the present invention.

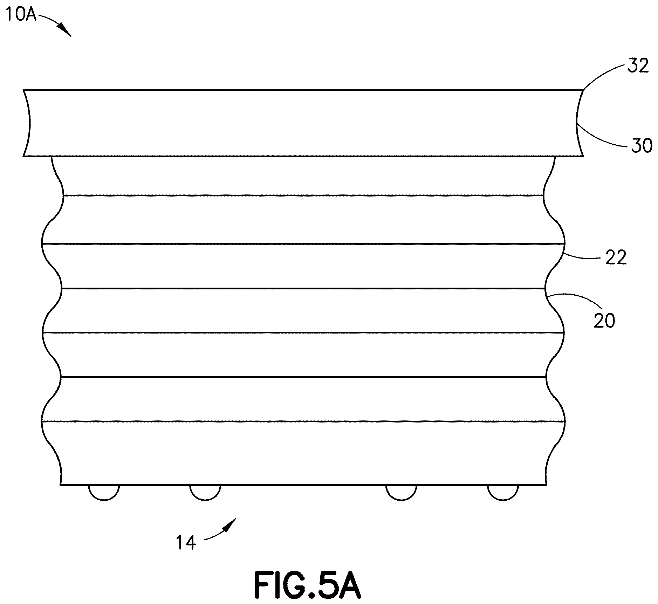

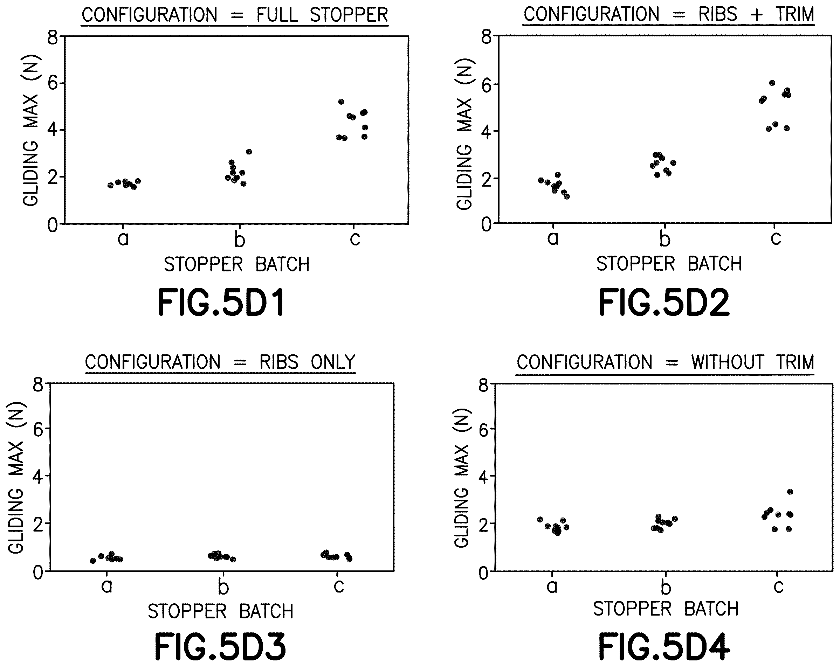

Fig. 5D2 is a graph showing the gliding performance of stopper batches a, b, and c wherein the distal closed front end of the stoppers have been removed in accordance with the configuration of Fig. 5A.

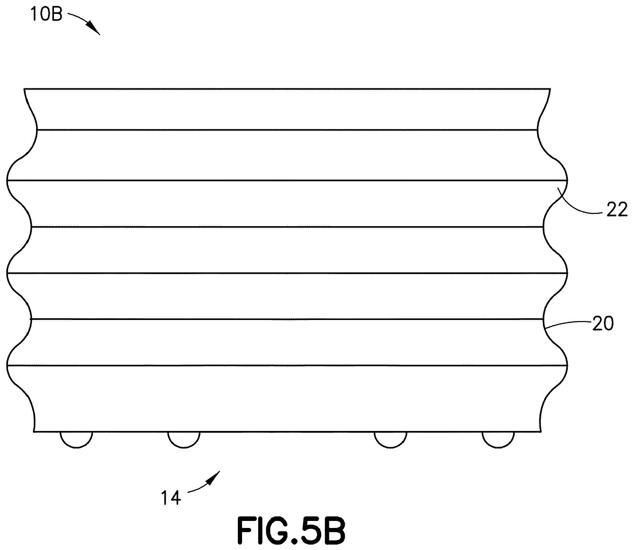

Fig. 5D3 is a graph showing the gliding performance of stopper batches a, b, and c wherein both the distal closed front end and the trim member of the stoppers have been removed in accordance with the configuration of Fig. 5B.

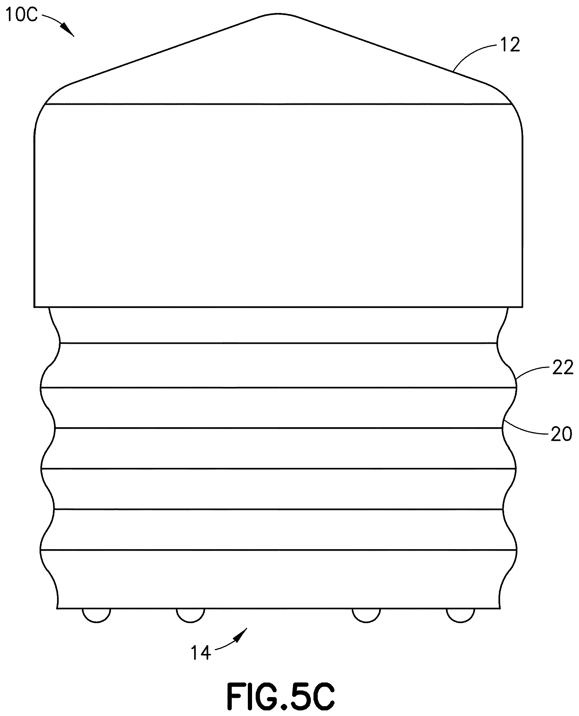

Fig. 5D4 is a graph showing the gliding performance of stopper batches a, b, and c wherein the stopper does not have a trim member in accordance with the configuration of Fig. 5C.

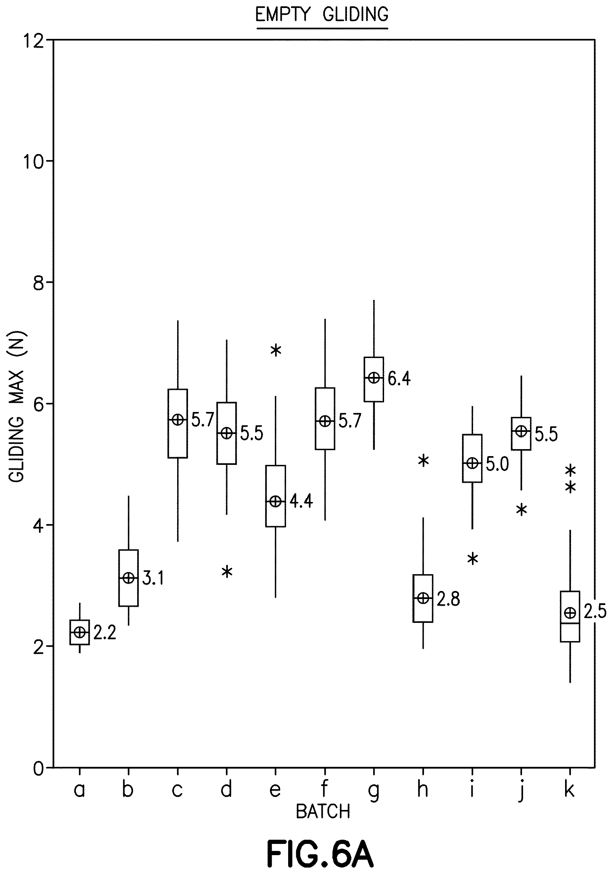

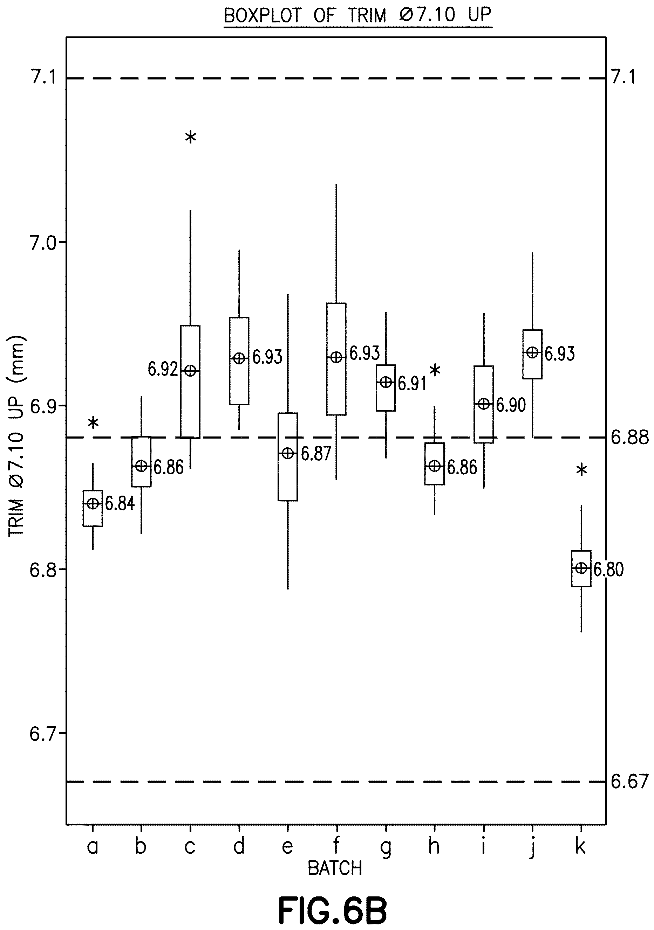

Figs. 6A and 6B are graphs illustrating the empty gliding performance and trim-up diameter of several batches of stoppers in accordance with an embodiment of the invention.

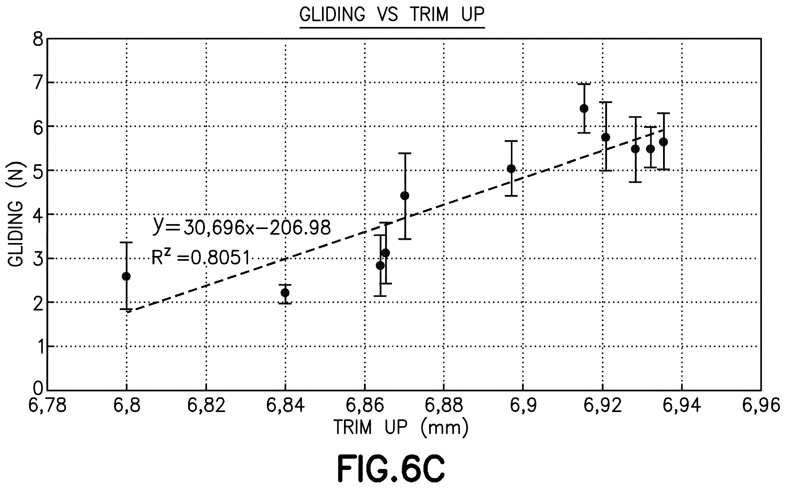

Fig. 6C is a graph illustrating the correlation between empty gliding and trim up diameter in accordance with an embodiment of the invention.

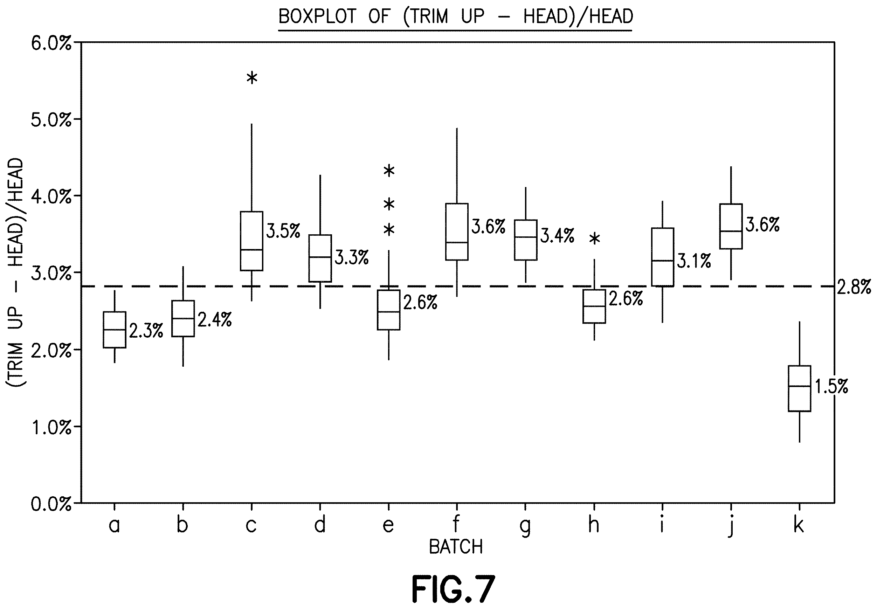

Fig. 7 is a graph illustrating the relative percentages of the diameter of the head of the stopper vs. the diameter of the trim-up-member in accordance with an embodiment of the invention.

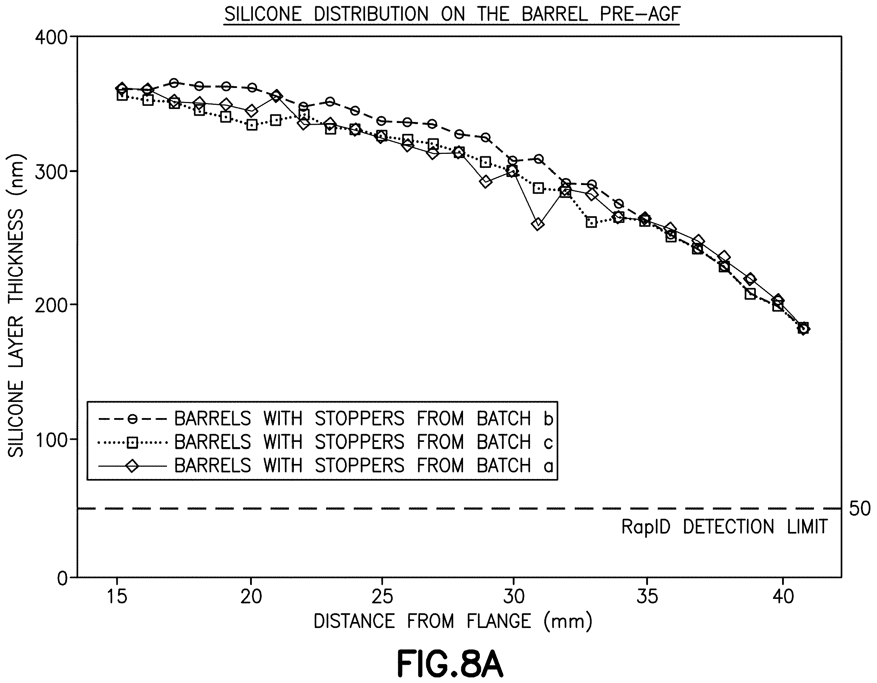

Fig. 8A is a graph illustrating the silicone distribution on the barrel pre-AGF (Activation and Gliding Force) test for batches a, b, and c in accordance with an embodiment of the invention.

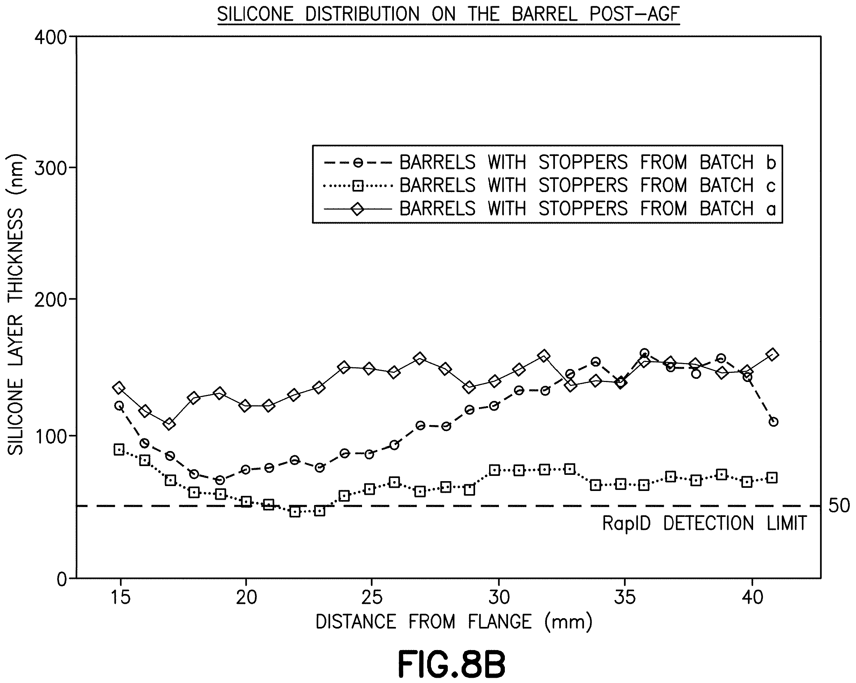

Fig. 8B is a graph illustrating the silicone distribution on the barrel post-AGF for batches a, b, and c in accordance with an embodiment of the invention.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the scope of the present invention.

For purposes of the description hereinafter, the terms "upper", "lower", "right", "left", "vertical", "horizontal", "top", "bottom", "lateral", "longitudinal", and derivatives thereof shall relate to the concept as it is oriented in the drawing figures. However, it is to be understood that the concept may assume various alternative variations, except where expressly specified to the contrary. It is also to be understood that the specific devices illustrated in the attached drawings, and described in the following specification, are simply exemplary embodiments of the concept. Hence, specific dimensions and other physical characteristics related to the embodiments disclosed herein are not to be considered as limiting.

Also, for purposes of the description of the present invention, the term "distal end" is intended to refer to the end of the syringe from which the needle projects and the end of the stopper which is closer to the syringe needle, whereas the term "proximal end" is intended to refer to the end of the syringe closer to the holder of the syringe and furthest from the needle tip and the end of the stopper furthest from the needle tip.

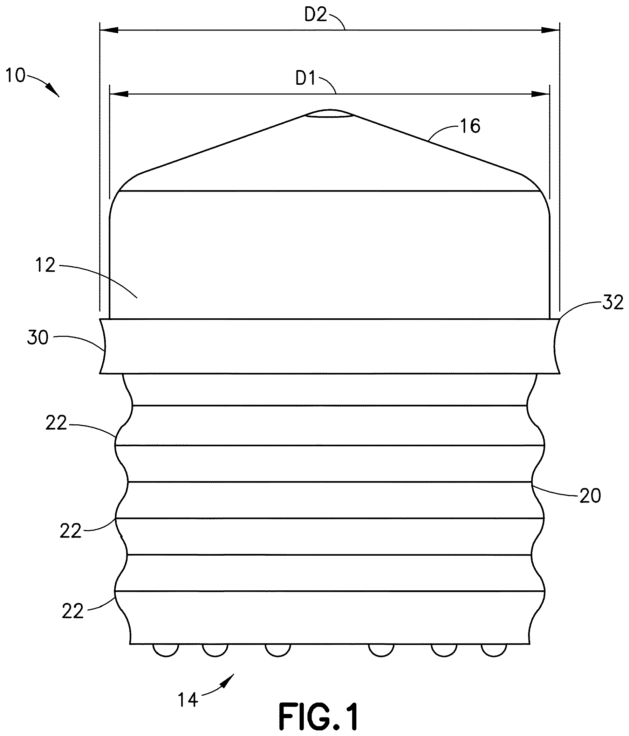

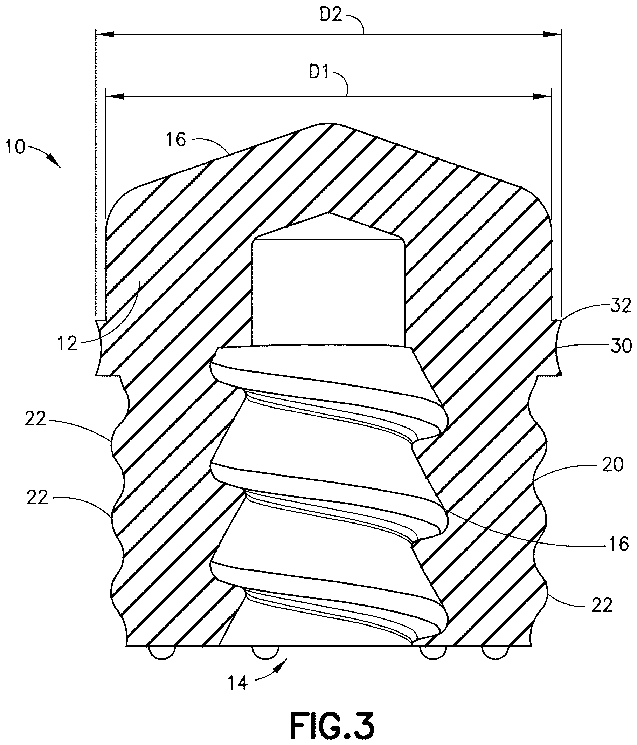

Reference is now made to Figs. 1-3 which shows a stopper, generally indicated as 10, adapted for attachment with a plunger rod (not shown) for use within a syringe barrel (not shown) in accordance with an embodiment of the invention. The stopper comprises a main body 12 defining an open rearward or proximal end 14, a closed front or distal end 16, and a cylindrical sidewall 20 extending between the open proximal end 14 and the closed distal end 16. The open proximal end 14 is adapted, such as via threads 16, shown in Fig. 3, to receive a front forward or distal forward end attachment portion (not shown) of the plunger rod. The stopper 10 further includes at least one rib 22, such as two or three ribs, preferably three ribs, extending radially outward around a perimeter of the main body 12. This at least one rib 22 is configured for forming an active seal with the syringe barrel. This at least one rib 22, along with the additional ribs of the stopper 10, are configured to ensure the seal of the stopper 10 with the barrel. The presence of several ribs ensures the container closure integrity. The stopper 10 further includes a trim member 30 extending radially outward around a perimeter of the main body 12. The trim member 30 is the location where individual stoppers 10 are trimmed from a molding sheet (not shown) during a trimming step. According to one embodiment, the stopper 10 can be molded in two parts and the trim member 30 is located at an interface of the closed distal end 16 and the open proximal end 14. The trim member 30 typically has the largest diameter of the stopper 10 and thus is in contact with the inner surface of the syringe barrel. The diameter of the trim member 30 is thus larger than the diameter of the inner surface of the syringe barrel. In accordance with the present invention, the trim member 30 is formed by controlling the geometry/shape, size, and/or diameter with respect to the diameter of the stopper 10 itself in order to reduce gliding forces of the stopper within the barrel. It can be appreciated that the stoppers and barrels can have different diameters depending upon the size of the syringe and/or injection device, as long as at least the stopper diameter ribs 22, and also preferably the trim member 30 are capable of forming an active seal with respect to the inner surface of the barrel so as to prevent liquid from seeping or escaping past the rearward or proximal end 14 of the stopper 10, thus ensuring the container closure integrity.

According to one embodiment, the trim member can be located between the closed distal end and the at least one rib, as shown in Figs. 1-3. A barrier film can be positioned onto or adjacent to the closed distal end 16. This barrier film can extend to a distally extending portion 32 of the trim member 30 and provides a barrier between the contents of the syringe and the stopper material. The material used for the barrier film can be ethylene tetrafluoroethylene (ETFE), however, it can be appreciated that other known types of materials exhibiting barrier properties can be used.

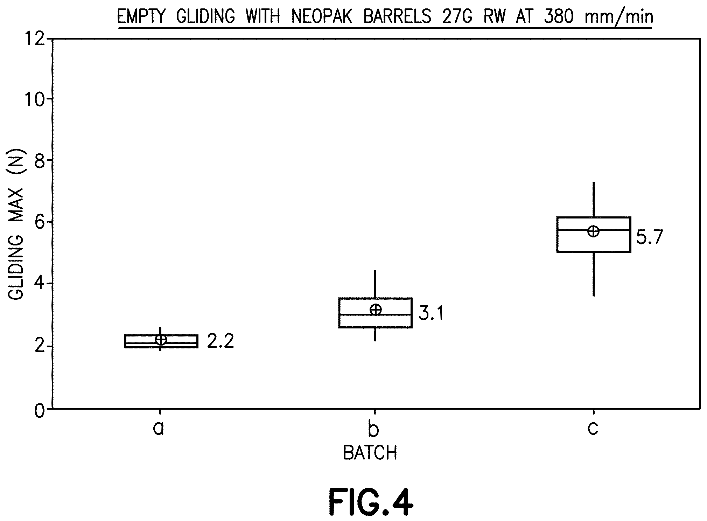

As discussed above, the invention is directed to an optimized stopper design through additional dimensional and geometry/shape control to produce a stopper 10 having lower gliding forces and to avoid the adverse impact of stress concentration typically generated by trimming processes. The gliding force (either for filled or empty syringes) is directly related to the main key product parameter of the syringe, i.e. the injection time of the drug contained in the syringe. This key product parameter is especially important for syringes used in auto-injectors, as these devices must comply with a defined injection time. However, prior to the present invention, some stopper designs, having trim members 30, showed a high variability regarding their gliding performances, as seen in Fig. 4, which shows gliding performances of three different batches, "a", "b", and "c" ranging from 2N up to 6N. Three different batches "a", "b", and "c" differ from each other with the dimension of the diameter of the distally extending portion 32 of the trim member. In that case, batch "a", "b", and "c" respectively have a diameter of the distally extending portion 32 of the trim member 30 of approximately 6.84mm, 6.86mm, and 6.92mm.

Prior to the present invention, certain stoppers, such as the one shown in Figs. 1-3 was produced in a single mold process, producing a laminated head having a barrier layer applied thereto, and several (i.e., 3) non-laminated ribs. The molding process would end with the individual stoppers being trimmed from the molding sheet. Oftentimes the trimming would occur between the laminated head and the back three ribs. An undesired effect of this process is that the trimmed width ends are the first place wherein the syringe contents will contact the stopper material without the barrier protection of the film head. This trimming width is often irregular and not controlled with respect to any particular dimensions, diameter, or shape.

In the present invention, testing has been done on three different stopper batches, batches "a", "b", and "c" to prove that the trim member 30 provides a significant contribution to the gliding performance of the stopper 10. Fig. 5A shows a stopper 10A comprised of both a trim member 30 and the ribs 22, but wherein the closed distal end 12 has been removed. Fig. 5B, shows a stopper 10B comprised of only the ribs 22, wherein the closed distal end 16 and trim member 30 have been removed. Fig. 5C, is a comparative stopper 10C having the closed distal end 16 and ribs 22, but does not have a trim member located thereon.

Fig. 5D shows a series of graphs, comparing each of the stoppers from batches "a", "b", and "c". The gliding force for the full stopper configuration (including a trim member), for batches "a", "b", and "c" is shown in graph 5D1. As can be seen in this graph, the stoppers have a gliding force similar to that shown in Fig. 4. The gliding force of stoppers from batches "a", "b", and "c", having the stopper configuration, as shown in Fig. 5A, with the three ribs 22 and a trim member 30, is shown in graph 5D2. The gliding force of stoppers from batches "a", "b", and "c", having the stopper configuration, as shown in Fig. 5B, of only the ribs 22, is shown in graph 5D3. The gliding force of the comparative stopper of batches "a", "b", and "c", configured as shown in Fig. 5C, where no trim member is present, is shown in graph 5D4. As can be seen from graphs 5D1 and 5D2, the full stopper having a rib member (graph 5D1) and the ribs plus trim configuration (graph 5D2) of Fig. 5B, have essentially the same gliding force. However, as can be seen in graph 5D3 and graph 5D4, batches "a", "b", and "c", where the trim member has been removed, do not show an appreciable difference in gliding force between the batches. This testing data proves that the trim member 30 is the main contributing factor in the differences in gliding performances.

Accordingly, the present invention has identified a relationship between the upper part or distally extending portion 32 of the trim member 30 and its functional performance (gliding) that has not been established or controlled before. This dimension can be referred to as a "Trim Up diameter" or D2, as discussed below.

Figs. 6A and 6B show the empty gliding performances (measure of the gliding force with an empty syringe) and the "Trim Up" dimensions of several stopper batches (a)-(k). A correlation can be made from these performances, as detailed in Fig. 6C. As a conclusion, to get lower and less variable gliding forces, a proposed specification for a "Trim Up" dimension can be an averaged maximum value of 6.67-6.88 mm and a maximum value of 6.91 mm. The head or distal end 16 of the stopper 10 can have a diameter of 6.75 +/- 0.08 mm, so that the maximum diameter can be approximately 6.83 mm. The internal diameter of the syringe barrel can be 6.35 +/- 0.1 mm. For other similar stopper designs, the specification can be applied on the gap between the "Trim Up" and the "head" diameter.

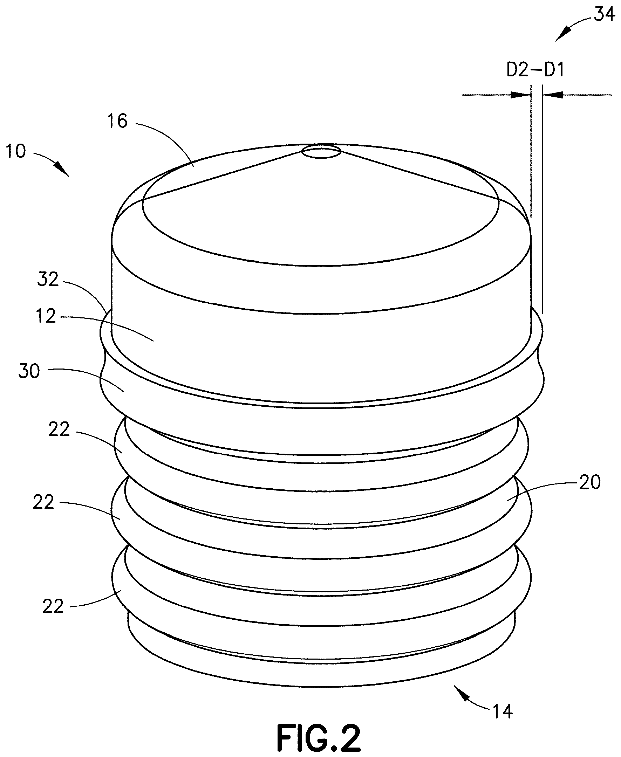

Referring back to Figs. 1 and 3, the stopper 10 has a first diameter D1 and the trim member 30 has a distally extending portion 32 having a second diameter D2, wherein the second diameter D2 is greater than the first diameter D1. According to one embodiment, the second diameter D2 is within 0.19 mm or less (of the first diameter D1, i.e. D2-D1, as shown by reference number 34 in Fig. 2. The second diameter of the distally extending portion 32 of the trim member 30 can be approximately 6.67- 6.91 mm.

Reference is now made to the graph of Fig. 7, which shows that the second diameter D2 can be approximately lower than 2.8% greater than the first diameter.

Accroding to the invention, this second diameter D2 can be approximately 2.3-2.8% greater than the first diameter D1.

In order to control the shape and/or geometry, and/or diameter of the trim member 30, the trim member 30 is formed with a cutting tool, as opposed to a molding process to form the stopper main body. The cutting tool can be a pre-stretched cutting die. Alternatively, the cutting tool can be a progressive cutting-edge die, which is a cutting tool that uses a wavy cutting edge engaging progressively on the rubber instead of a one-cut stroke die, which is prone to rubber displacement due to compressive stresses and the Poisson coupling effect. It can be appreciated that other types of cutting tools can be used to separate the stopper from the molding sheet.

In accordance with another aspect and with reference back to Figs. 1-3 and Figs. 6A-7, the present invention is directed to a method for forming consistent gliding forces for a series of stoppers 10 adapted for attachment with a series of plunger rods for use in a series of syringe barrels. The method comprises providing a series of stoppers 10, each of the stoppers comprising a main body 12, defining an open rearward or proximal end 14, a closed front or distal end 16, and a cylindrical sidewall 20 extending between the open rearward end 14 and the closed front end 16. The open proximal end 14 is adapted to receive a front distal end attachment portion (not shown) of the plunger rod. At least one rib 22 is provided that extends radially outward around a perimeter of the main body 12. The at least one rib 22 can comprise three ribs, and be configured for forming an active seal with the syringe barrel. A trim member 30 extends radially outward around a perimeter of main body 12. The trim member 30 includes a distally extending portion 32 having a relative diameter configured for reducing gliding forces of the stopper within the barrel, wherein a width of the trim member 30 of each of the series of stoppers is controlled by using a cutting tool. The cutting tool can be one of a pre-stretched cutting die or a progressive cutting-edge die. Other known cutting dies can be used for cutting the trim member wherein the shape, geometry, and/or diameter of the trim member is controlled. The use of a cutting tool enables closer control and tolerances of the trim rib dimensions and shape, resulting in a controlled and consistent trim member 30.

Each of the stoppers 10 are designed to have a first diameter D1 and the trim member 30 of each of the stoppers 10 are designed to have a distally extending portion 32 having a second diameter D2, wherein the second diameter D2 is greater than the first diameter D1. According to the invention, this second diameter D2 can be approximately 2.3-2.8% greater than the first diameter D1. The method can further include applying a barrier film on and/or adjacent to the closed distal end 12 of each of the stoppers 10, such that the barrier film extends to the trim member 30. The method further comprises removing the excess barrier material from the stoppers 10.

Referring now to Figs. 8A and 8B, it has been found, in the present invention, that one physical phenomenon explaining the correlation between the "Trim Up" dimensions and the gliding force is the wiping of the silicon layer from the barrel. This is due to the concentration of stresses caused by the shape of the "Trim Up". The bigger the gap, as shown by 34 in Fig. 2, between the trim member 30 and the head or front end 12 of the stopper 10, the more lubricant, i.e., silicone, is scraped from the barrel. This is because there is a larger compression ratio at the "Trim Up" and the corresponding contact pressure. Up to a threshold, the less silicone in the barrel, the higher the gliding force. This phenomenon is also related to the viscosity of the silicone oil and how fast it is squeezed out from the "Trim Up" contact area. The higher the silicone oil viscosity, the less it is squeezed out. However, the higher viscosity will mean that there is a penalty in gliding due to Reynolds lubrication. It is expected that the penalty in gliding force for the increased viscosity is minor compared to the squeeze out effect due to high contact pressure. This is shown in Figs. 8A and 8B where the silicone distribution has been analyzed before and after the gliding test on the stoppers from batches "a", "b", and "c". In the test, fifteen stoppers (five from 3 different batches) have been tested for empty AGF (Activation Gliding Force). Measures of the silicone layer thickness have also been performed. Before the AGF test, the fifteen barrels used were homogeneous regarding their silicone distribution. The results are shown in Fig. 8A.. Post-AGF testing, as shown in Fig. 8B shows that the barrels from Batch "c", which corresponds to the stopper with the higher gliding force, as shown in Fig. 4 (approximately 5.7N), shows a lower silicone distribution than the other barrels and indicates that the stopper from batch "c" removed more silicone from the barrel than the other stopper batches.

While the disclosure has been described as having exemplary designs, the present disclosure can be further modified within the scope of this disclosure. This application is, therefore, intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A stopper (10, 10A, 10B, 10C) adapted for attachment with a plunger rod for use within a syringe barrel, said stopper comprising:
a main body (12) defining an open proximal end (14), a closed distal end (16), and a cylindrical sidewall (20) extending between the open proximal end and the closed distal end, said open proximal end adapted to receive a distal forward end attachment portion of the plunger rod;
at least one rib (22) extending radially outward around a perimeter of said main body, said at least one rib configured for forming an active seal with the syringe barrel; and
a trim member (30) extending radially outward around a perimeter of said main body at an interface between the closed distal end and the open proximal end, said trim member having a shape, size, and/or diameter configured for reducing gliding force of the stopper within the barrel, **characterised in that** the stopper closed distal end (16) has a first diameter (D1) and the trim member has a distally extending portion having a second diameter (D2), wherein the second diameter is approximately 2.3-2.8% greater than the first diameter.

2. The stopper of claim 1, wherein the stopper is molded in two parts and the trim member is located between the closed distal end of the stopper and the at least one rib.

3. The stopper of any of claims 1 or 2 including a barrier film positioned on and/or adjacent the closed distal end.

4. A method for forming consistent gliding forces for a series of stoppers, adapted for attachment with a series of plunger rods for use within a series of syringe barrels, said method comprising:
providing a series of stoppers (10, 10A, 10B, 10C), each of said stoppers comprising a main bod: (12) defining an open proximal end (14), a closed distal end (16), and a cylindrical sidewall (20) extending between the open proximal end and the closed distal end, said open proximal end adapted to receive a front distal end attachment portion of the plunger rod; at least one rib (22) extending radially outward around a perimeter of said main body, said at least one rib configured for forming an active seal with the syringe barrel; and a trim member (30) extending radially outward around a perimeter of said main body, said trim member having a shape, size, and/or diameter configured for reducing gliding forces of the stoppers within the barrel, wherein the shape, size, and/or diameter of the trim member of each of the series of stoppers is controlled by using a cutting tool, **characterised in that** each of the stopper have a first diameter (D1) and the trim member of each of the stoppers has a distal extending portion having a second diameter (D2), wherein the second diameter is approximately 2.3-2.8% greater than the first diameter..

5. The method of claim 4, comprising applying a barrier film on and/or adjacent to the closed front end of each of the stoppers, such that the barrier film extends to the trim member, and removing any excess barrier material from each of the stoppers.

6. The method of any of claims 4 or 5, wherein the cutting tool comprises a pre-stretched cutting die or a progressive cutting-edge die.

## Patentansprüche

1. Stopper (10, 10A, 10B, 10C), der zum Befestigen mit einer Kolbenstange zur Verwendung innerhalb eines Spritzenzylinders angepasst ist, wobei der Stopper umfasst:
einen Hauptkörper (12), der ein offenes proximales Ende (14), ein geschlossenes distales Ende (16) und eine zylindrische Seitenwand (20), die sich zwischen dem offenen proximalen Ende und dem geschlossenen distalen Ende erstreckt, definiert, wobei das offene proximale Ende angepasst ist, um einen distalen vorderen Endbefestigungsabschnitt der Kolbenstange aufzunehmen;
mindestens eine Rippe (22), die sich radial nach außen um einen Umfang des Hauptkörpers erstreckt, wobei die mindestens eine Rippe zum Bilden einer aktiven Abdichtung mit dem Spritzenzylinder konfiguriert ist; und
ein Verkleidungselement (30), das sich radial nach außen um einen Umfang des Hauptkörpers an einer Schnittstelle zwischen dem geschlossenen distalen Ende und dem offenen proximalen Ende erstreckt, wobei das Verkleidungselement eine Form, Größe und/oder einen Durchmesser aufweist, die/der konfiguriert ist, um die Gleitkraft des Stoppers innerhalb des Zylinders zu reduzieren,
**dadurch gekennzeichnet, dass** das geschlossene distale Ende (16) des Stoppers einen ersten Durchmesser (D1) aufweist und das Verkleidungselement einen sich distal erstreckenden Abschnitt aufweist, der einen zweiten Durchmesser (D2) aufweist, wobei der zweite Durchmesser annähernd 2,3-2,8% größer als der erste Durchmesser ist.

2. Stopper nach Anspruch 1, wobei der Stopper in zwei Teilen geformt ist und sich das Verkleidungselement zwischen dem geschlossenen distalen Ende des Stoppers und der mindestens einen Rippe befindet.

3. Stopper nach einem der Ansprüche 1 oder 2, der eine Barrierefolie beinhaltet, die auf und/oder neben dem geschlossenen distalen Ende positioniert ist.

4. Verfahren zum Bilden gleichmäßiger Gleitkräfte für eine Reihe von Stoppern, die zum Befestigen mit einer Reihe von Kolbenstangen zur Verwendung innerhalb einer Reihe von Spritzenzylindern angepasst sind, wobei das Verfahren umfasst:
Bereitstellen einer Reihe von Stoppern (10, 10A, 10B, 10C), wobei jeder der Stopper einen Hauptkörper (12) umfasst, der ein offenes proximales Ende (14), ein geschlossenes distales Ende (16) und eine zylindrische Seitenwand (20), die sich zwischen dem offenen proximalen Ende und dem geschlossenen distalen Ende erstreckt, definiert, wobei das offene proximale Ende angepasst ist, um einen vorderen distalen Endbefestigungsabschnitt der Kolbenstange aufzunehmen; mindestens eine Rippe (22), die sich radial nach außen um einen Umfang des Hauptkörpers erstreckt, wobei die mindestens eine Rippe zum Bilden einer aktiven Abdichtung mit dem Spritzenzylinder konfiguriert ist; und ein Verkleidungselement (30), das sich radial nach außen um einen Umfang des Hauptkörpers erstreckt, wobei das Verkleidungselement eine Form, Größe und/oder einen Durchmesser aufweist, die zum Reduzieren von Gleitkräften der Stopper innerhalb des Zylinders konfiguriert sind, wobei die Form, Größe und/oder der Durchmesser des Verkleidungselements jedes der Reihe von Stoppern durch Verwendung eines Schneidwerkzeugs gesteuert wird, **dadurch gekennzeichnet, dass** jeder der Stopper einen ersten Durchmesser (D1) aufweist und das Verkleidungselement jedes der Stopper einen sich distalen erstreckenden Abschnitt aufweist, der einen zweiten Durchmesser (D2) aufweist,
wobei der zweite Durchmesser annähernd 2,3-2,8% größer als der erste Durchmesser ist.

5. Verfahren nach Anspruch 4, umfassend Aufbringen einer Barrierefolie auf und/oder neben dem geschlossenen vorderen Ende jedes der Stopper, sodass sich die Barrierefolie bis zum Verkleidungselement erstreckt, und Entfernen überschüssigen Barrierematerials von jedem der Stopper.

6. Verfahren nach einem der Ansprüche 4 oder 5, wobei das Schneidwerkzeug eine vorgestreckte Schneidmatrize oder eine progressive Schneidkantenmatrize umfasst.

## Revendications

1. Bouchon (10, 10A, 10B, 10C) adapté pour être fixé à une tige de piston pour une utilisation à l'intérieur d'un cylindre de seringue, ledit bouchon comprenant :
un corps principal (12) définissant une extrémité proximale ouverte (14), une extrémité distale fermée (16) et une paroi latérale cylindrique (20) s'étendant entre l'extrémité proximale ouverte et l'extrémité distale fermée, ladite extrémité proximale ouverte étant adaptée pour recevoir une partie de fixation d'extrémité avant distale de la tige de piston ;
au moins une nervure (22) s'étendant radialement vers l'extérieur autour d'un périmètre dudit corps principal,
ladite au moins une nervure étant configurée pour former un joint actif avec le cylindre de seringue ; et
un élément de garniture (30) s'étendant radialement vers l'extérieur autour d'un périmètre dudit corps principal au niveau d'une interface entre l'extrémité distale fermée et l'extrémité proximale ouverte, ledit élément de garniture ayant une forme, une taille et/ou un diamètre configurés pour réduire la force de glissement du bouchon à l'intérieur du cylindre,
**caractérisé en ce que** l'extrémité distale fermée (16) du bouchon a un premier diamètre (D1) et l'élément de garniture a une partie s'étendant de manière distale ayant un second diamètre (D2), dans lequel le second diamètre est supérieur d'environ 2,3 à 2,8% au premier diamètre.

2. Bouchon selon la revendication 1, dans lequel le bouchon est moulé en deux parties et l'élément de garniture est situé entre l'extrémité distale fermée du bouchon et l'au moins une nervure.

3. Bouchon selon l'une des revendications 1 ou 2, comprenant un film barrière positionné sur et/ou de manière adjacente à l'extrémité distale fermée.

4. Procédé pour former des forces de glissement constantes pour une série de bouchons, adaptés pour être fixés à une série de tiges de piston pour une utilisation dans une série de cylindres de seringues, ledit procédé comprenant :
la fourniture d'une série de bouchons (10, 10A, 10B, 10C), chacun desdits bouchons comprenant un corps principal (12) définissant une extrémité proximale ouverte (14), une extrémité distale fermée (16) et une paroi latérale cylindrique (20) s'étendant entre l'extrémité proximale ouverte et l'extrémité distale fermée, ladite extrémité proximale ouverte étant adaptée pour recevoir une partie de fixation d'extrémité distale avant de la tige de piston ; au moins une nervure (2) s'étendant radialement vers l'extérieur autour d'un périmètre dudit corps principal, ladite au moins une nervure étant configurée pour former un joint actif avec le cylindre de seringue ; et un élément de garniture (30) s'étendant radialement vers l'extérieur autour d'un périmètre dudit corps principal, ledit élément de garniture ayant une forme, une taille et/ou un diamètre configurés pour réduire les forces de glissement des bouchons à l'intérieur du cylindre, dans lequel la forme, la taille et/ou le diamètre de l'élément de garniture de chacun de la série de bouchons sont réglés en utilisant un outil de coupe, **caractérisé en ce que** chacun des bouchons a un premier diamètre (D1) et l'élément de garniture de chacun des bouchons a une partie s'étendant de manière distale ayant un second diamètre (D2), dans lequel le second diamètre est supérieur d'environ 2,3 à 2,8% au premier diamètre.

5. Procédé selon la revendication 4, comprenant l'application d'un film barrière sur et/ou de manière adjacente à l'extrémité avant fermée de chacun des bouchons, de sorte que le film barrière s'étende jusqu'à l'élément de garniture, et l'élimination de tout excès de matériau barrière de chacun des bouchons.

6. Procédé selon l'une des revendications 4 ou 5, dans lequel l'outil de coupe comprend une matrice de coupe préétirée ou une matrice à arête de coupe progressive.
